# EUROPEAN PATENT SPECIFICATION

(11) **EP 1 868 627 B1**
(45) Date of publication and mention of the grant of the patent: **15.10.2014**
(21) Application number: 06726589.2
(22) Date of filing: 30.03.2006
(51) Int. Cl.: A61K 38/23, A61P 19/02

(54) **USE OF CALCITONIN AS COMBINED TREATMENT THERAPY FOR THE MANAGEMENT OF INFLAMMATORY DISEASE CONDITIONS**
VERWENDUNG VON CALCITONIN ALS KOMBINIERTE THERAPIE ZUR BEHANDLUNG VON ENTZÜNDLICHEN ERKRANKUNGEN
UTILISATION DE LA CALCITONINE EN TANT QUE TRAITEMENT THERAPEUTIQUE COMBINE A DES FINS DE GESTION D'ETATS PATHOLOGIQUES INFLAMMATOIRES

(30) Priority: 01.04.2005 GB 0506708
(43) Date of publication of application: 26.12.2007
(73) Proprietor: QUEEN MARY AND WESTFIELD COLLEGE, London E1 4NS (GB)
(72) Inventor: MACINTYRE, Ian, William Harvey Research Institute, London EC1M 6BQ (GB); PERRETTI, Mauro, William Harvey Research Institute, London EC1M 6BQ (GB); MANCINI, Lucia, 51100 Pistoia (IT)
(74) Representative: Bassil, Nicholas Charles
(86) International application number: PCT/GB2006/001183
(87) International publication number: WO 2006/103455

(56) References cited:
- WO-A2-01/13957
- WO-A2-2005/009949
- DATABASE BIOSIS [Online] BIOSCIENCES INFORMATION SERVICE, PHILADELPHIA, PA, US; 1987, BADURSKI J ET AL: "THERAPEUTIC USE OF CALCITONIN IN CONCOMITANT DISEASES OF THE JOINTS AND STOMACH II. CLINICAL ASSESSMENT OF CALCITONIN EFFECT ON CHRONIC PEPTIDE ULCER IN PATIENTS WITH RHEUMATOID ARTHRITIS AND DEGENERATIVE JOINT DISEASE" XP002405253 Database accession no. PREV198987008753 & REUMATOLOGIA (WARSAW), vol. 25, no. 1, 1987, pages 17-23, ISSN: 0034-6233
- DATABASE BIOSIS [Online] BIOSCIENCES INFORMATION SERVICE, PHILADELPHIA, PA, US; 1986, BADURSKI J ET AL: "THERAPEUTIC USE OF CALCITONIN IN CONCOMITANT DISEASES OF THE JOINTS AND STOMACH" XP002405254 Database accession no. PREV198886084069 & REUMATOLOGIA (WARSAW), vol. 24, no. 4, 1986, pages 284-288, ISSN: 0034-6233
- DATABASE BIOSIS [Online] BIOSCIENCES INFORMATION SERVICE, PHILADELPHIA, PA, US; 1984, BONAVITA E: "SALMON CALCITONIN-ANTIINFLAMMATORY COMBINATION IN MANAGEMENT OF PAINFUL SPONDYLOARTHRITIS CLINICAL AND EXPERIMENTAL STUDY" XP002405255 Database accession no. PREV198580006465 & CLINICA TERAPEUTICA, vol. 110, no. 2, 1984, pages 123-128, ISSN: 0009-9074
- GOLDSTEIN MARC F ET AL: "Chronic glucocorticoid therapy-induced osteoporosis in patients with obstructive lung disease" CHEST, vol. 116, no. 6, December 1999 (1999-12), pages 1733-1749, XP002405245 ISSN: 0012-3692
- GOSPODINOFF A ET AL: "CALCITONIN IN TREATMENT OF COXARTHROSIS LA CALCITONINA NEL TRATTAMENTO DELLA COXARTROSI" CLINICA TERAPEUTICA, SOCIETA EDITRICE UNIVERSO, ROME, IT, vol. 110, no. 2, 1984, pages 129-133, XP009037568 ISSN: 0009-9074
- CONSOLI G ET AL: "CALCITONIN IN RHEUMATOLOGIE OSTEOPOROSIS ASPECTS AND PRELIMIN EVALUATION OF PATHOPHYSIOLOGY AND CLINICAL FEATURES" CLINICA TERAPEUTICA, SOCIETA EDITRICE UNIVERSO, ROME, IT, vol. 118, no. 1, 1996, pages 37-47, XP008016118 ISSN: 0009-9074
- BECKER K L ET AL: "Clinical review 167 - Procalcitonin and the calcitonin gene family of peptides in inflammation, infection, and sepsis: A journey from calcitonin back to its precursors" JOURNAL OF CLINICAL ENDOCRINOLOGY & METABOLISM, vol. 89, no. 4, April 2004 (2004-04), pages 1512-1525, XP002405246 ISSN: 0021-972X
- BINSTOCK M L ET AL: "EFFECT OF CALCITONIN AND GLUCO CORTICOIDS IN COMBINATION ON THE HYPER CALCEMIA OF MALIGNANCY", ANNALS OF INTERNAL MEDICINE, vol. 93, no. 2, 1980, pages 269-272, ISSN: 0003-4819
- Rahnama M., et al.: "[The influence of experimental corticoid therapy on the zinc levels in the rat's teeth].[Article in Polish]", PUBMED Abstract Rocz Panstw Zakl Hig., vol. 52, no. 2 2001, pages 119-125, Retrieved from the Internet: URL:http://www.ncbi.nlm.nih.gov/pubmed/115 56096?dopt=Abstractrecent.wl.history.lengt h%20=%205 [retrieved on 2012-05-08]
- HEALEY J H ET AL: "A randomized controlled trial of salmon calcitonin to prevent bone loss in corticosteroid-treated temporal arteritis and polymyalgia rheumatica", CALCIFIED TISSUE INTERNATIONAL 1996 US LNKD- DOI:10.1007/S002239900014, vol. 58, no. 2, 1996, pages 73-80, XP008151723, ISSN: 0171-967X
- MONTEMURRO L ET AL: "Prevention of corticosteroid-induced osteoporosis with salmon calcitonin in sarcoid patients", CALCIFIED TISSUE INTERNATIONAL 1991 US, vol. 49, no. 2, 1991, pages 71-76, XP008151724, ISSN: 0171-967X
- LUENGO M. ET AL.,: 'Prevention of further bone mass loss by nasal calcitonin in patients on long term glucocorticoid therapy for asthma: a two year follow up study' THORAX vol. 49, 1994, pages 1099 - 1102

## Description

### FIELD OF THE INVENTION

The present invention relates to the use of calcitonin and a glucocorticoid as a combined treatment therapy for the management of inflammatory pathologies and arthritides.

### BACKGROUND OF THE INVENTION

Glucocorticoid drugs (GCs) are potent immunosuppressive and anti-inflammatory agents that are therapeutically used in several inflammatory pathologies. Long-term therapy with GCs is often necessary to control the symptoms of rheumatoid arthritis and other rheumatic conditions. Recent evidence shows that GCs may have a disease-modifying effect in addition to their well-documented anti-inflammatory actions. However, therapeutic management of long-term pathologies with GC is often linked to a series of unwanted side effects, involving the hypothalamus-pituitary-adrenal axis, the cardiovascular system as well as fat and bone metabolism. In particular, secondary osteoporosis is one of the major problems associated with long term GC therapy in rheumatoid arthritis patients. Several mechanisms are implicated in GC-induced bone loss including direct effects on bone tissue cells and modulation of cytokine and growth factor production.

Calcitonin (CT or Ct) is a peptide hormone best known for its anti-osteoclastic activity and is a member of a family of proteins with homologues identified in most species studied to date. The Calcitonins (Cts) are peptide hormones of 32 amino-acid residues that have been mainly known for their hypocalcemic effect and the inhibition of bone-resorption. Calcitonins are used therapeutically for the treatment of osteoporosis and other with bone disorder-related diseases. Salmon Ct (sCt) has been shown to have the greatest pharmaceutical activity in the treatment of human disease conditions, whereas the human hormone (hCt) has a strongly reduced potency when administered in a therapeutic setting. As a result, salmon calcitonin (sCt) is the main Ct to be applied therapeutically to date. However, there is only a 50% sequence homology between sCt and hCt, which is the cause for immunogenic reactions in humans when treated with sCt.

The current state of therapy of rheumatoid arthritis has been dramatically improved by the use of newer biological therapies that have reduced dependence of steroids and non-steroidal agents. One of the most widely used of these therapies, for example, is Enbrel™, a construct of the TNF-α receptor coupled to immunoglobulin. Although quite effective the newer biological therapies have not completely suppressed but only significantly modified the symptoms. Moreover these new drugs are very expensive.

Healy et al, Calcif Tissue Int. 58:73-80 (1996) relates to a randomized controlled trial of salmon calcitonin to prevent bone loss in corticosteroid-treated temporal arteritis and polymyalgia rheumatica.

Montemurro et al, Calcif. Tissue Int. 49:71-76 (1991) relates to the prevention of corticosteroid-induced osteoporosis with salmon calcitonin in sarcoid patients.

Luengo et al, Thorax 49:1099-1102 (1994) relates to a two year follow up study on the prevention of further bone mass loss by nasal calcitonin in patients on long term glucocorticoid therapy for asthma.

Binstock et al, Ann. Int. Med. 93 : 269-272 (1980) relates to the prevention of hypercalcemia with the combination of calcitonin and glucocorticoids (prednisone) in patients with malignant disease.

For these reasons glucocorticoids (GC) are still widely used as potent immuno-suppressive and anti-inflammatory agents in all forms of chronic inflammation, including rheumatoid arthritis. However, as stated above, long-term therapies with GC are associated with secondary osteoporosis, hence, the beneficial anti-inflammatory action of GC can be impaired by an exacerbating effect on bone erosion.

### SUMMARY OF THE INVENTION

According to a first aspect of the invention, there is provided calcitonin for use in the treatment of an inflammatory disease or condition in combination with a glucocorticoid, wherein the inflammatory disease or conditions is selected from the group consisting of arthritis, rheumatoid arthritis (RA), juvenile rheumatoid arthritis (JRA), osteoarthritis, psoriasis, psoriatic arthritis, ankylosing spondylitis (AS), lupus erythamatosis, multiple sclerosis (MS), asthma, and immune-suppressive therapy.

The calcitonin may be from any convenient source, including both isolated natural forms of the hormone, or synthetic, e.g. from a recombinant source. The calcitonin peptide may be selected from the group consisting of human, salmon, porcine, bovine, eel or chicken calcitonin or an active fragment or derivative thereof. Calcitonin peptides include but are not limited to peptides from the following sources:

Cys-Ser-Asn-Leu-Ser-Thr-Cys-Val-Leu-Ser-Ala-Tyr-Trp-Arg-Asn-Leu-Asn-Asn-Phe-His-
Arg-Phe-Ser-Gly-Met-Gly-Phe-Gly-Pro-Glu-Thr-Pro (Porcine)

Cys-Ser-Asn-Leu-Ser-Thr-Cys-Val-Leu-Ser-Ala-Tyr-Trp-Lys-Asp-Leu-Asn-Asn-Tyr-His-
Arg-Phe-Ser-Gly-Met-Gly-Phe-Gly-Pro-Glu-Thr-Pro (Bovine)

Cys-Ser-Asn-Leu-Ser-Thr-Cys-Val-Leu-Gly-Lys-Leu-Ser-Gln-Glu-Leu-His-Lys-Leu-Gln-
Thr-Tyr-Pro-Arg-Thr-Asn-Thr-Gly-Ser-Gly-Thr-Pro (Salmon)

Cys-Ser-Asn-Leu-Ser-Thr-Cys-Val-Leu-Gly-Lys-Leu-Ser-Gln-Glu-Leu-His-Lys-Leu-Gln-
Thr-Tyr-Pro-Arg-Thr-Asp-Val-Gly-Ala-Gly-Thr-Pro (Eel)

Cys-Gly-Asn-Leu-Ser-Thr-Cys-Met-Leu-Gly-Thr-Tyr-Thr-Gln-Asp-Phe-Asn-Lys-Phe-His-
Thr-Phe-Pro-Gln-Thr-Ala-Leu-Gly-Val-Gly-Ala-Pro (Human)

Cys-Ala-Ser-Leu-Ser-Thr-Cys-Val-Leu-Gly-Lys-Leu-Ser-Gln-Glu-Leu-His-Lys-Leu-Gln-
Thr-Tyr-Pro-Arg-Thr-Asp-Val-Gly-Ala-Gly-Thr-Pro (Chicken)

The calcitonins used according to the present invention also include variants, fragments and/or derivatives of a calcitonin peptide.

An example of a variant of the present invention is a fusion protein comprising a calcitonin peptide as defined above, apart from the substitution of one or more amino acids with one or more other amino acids. The skilled person is aware that various amino acids have similar properties. One or more such amino acids of a substance can often be substituted by one or more other such amino acids without eliminating a desired activity of that substance.

Thus the amino acids glycine, alanine, valine, leucine and isoleucine can often be substituted for one another (amino acids having aliphatic side chains). Of these possible substitutions it is preferred that glycine and alanine are used to substitute for one another (since they have relatively short side chains) and that valine, leucine and isoleucine are used to substitute for one another (since they have larger aliphatic side chains which are hydrophobic). Other amino acids which can often be substituted for one another include: phenylalanine, tyrosine and tryptophan (amino acids having aromatic side chains); lysine, arginine and histidine (amino acids having basic side chains); aspartate and glutamate (amino acids having acidic side chains); asparagine and glutamine (amino acids having amide side chains); and cysteine and methionine (amino acids having sulphur containing side chains).

Substitutions of this nature are often referred to as "conservative" or "semi-conservative" amino acid substitutions.

Amino acid deletions or insertions may also be made relative to the amino acid sequence for the fusion protein referred to above. Thus, for example, amino acids which do not have a substantial effect on the activity of the polypeptide, or at least which do not eliminate such activity, may be deleted. Such deletions can be advantageous since the overall length and the molecular weight of a polypeptide can be reduced whilst still retaining activity. This can enable the amount of polypeptide required for a particular purpose to be reduced - for example, dosage levels can be reduced.

Amino acid insertions relative to the sequence of the fusion protein above can also be made. This may be done to alter the properties of a substance of the present invention (e.g. to assist in identification, purification or expression, as explained above in relation to fusion proteins).

Amino acid changes relative to the sequence given above can be made using any suitable technique e.g. by using site-directed mutagenesis or solid state synthesis...

It should be appreciated that amino acid substitutions or insertions within the scope of the present invention can be made using naturally occurring or non-naturally occurring amino acids. Whether or not natural or synthetic amino acids are used, it is preferred that only L- amino acids are present.

Some preferred substitutions may be for basic and/or acidic amino acid residues with neutral amino acid residues. Other substitutions may be the replacement of the C-terminal residue with homoserine (Hse).

Examples of derivatives include but are not limited to the formation of intramolecular disulfide bonds between cysteine residues in the peptide or other modifications so as to form a cyclic or a ring-shaped structure. Other post-translational modifications may also be made such as phosphorylation, glycosylation, N-terminal amidation etc. Glycosylation may be the addition of a sugar residue such as N-acetyl-D-glucosamine moiety.

Fragments of the calcitonin peptide include calcitonin peptides that a truncated by deletion of amino acid residues 1 to 9. However, it may be preferred that residues 17 to 21 at least are present in the calcitonin fragment.

The sequences of the fragments truncated by deletion of residues 1 to 9 are as follows:

Ser-Ala-Tyr-Trp-Arg-Asn-Leu-Asn-Asn-Phe-His-Arg-Phe-Ser-Gly-Met-Gly-Phe-Gly-Pro-
Glu-Thr-Pro (Porcine)

Ser-Ala-Tyr-Trp-Lys-Asp-Leu-Asn-Asn-Tyr-His-Arg-Phe-Ser-Gly-Met-Gly-Phe-Gly-Pro-
Glu-Thr-Pro (Bovine)

Gly-Lys-Leu-Ser-Gln-Glu-Leu-His-Lys-Leu-Gln-Thr-Tyr-Pro-Arg-Thr-Asn-Thr-Gly-Ser-
Gly-Thr-Pro (Salmon)

Gly-Lys-Leu-Ser-Gln-Glu-Leu-His-Lys-Leu-Gln-Thr-Tyr-Pro-Arg-Thr-Asp-Val-Gly-Ala-
Gly-Thr-Pro (Eel)

Gly-Thr-Tyr-Thr-Gln-Asp-Phe-Asn-Lys-Phe-His-Thr-Phe-Pro-Gln-Thr-Ala-Leu-Gly-Val-
Gly-Ala-Pro (Human)

Gly-Lys-Leu-Ser-Gln-Glu-Leu-His-Lys-Leu-Gln-Thr-Tyr-Pro-Arg-Thr-Asp-Val-Gly-Ala-
Gly-Thr-Pro (Chicken)

Preferred peptides for use according to the present invention are salmon calcitonin (sCt), eel calcitonin (eCt) and human calcitonin (hCt) from either natural or recombinant sources, or an active fragment thereof, for example a (1-9)fragment as described above.

Treatment of the inflammatory disease or condition may include prophylaxis as well as treatment of the disease. Treatment may be of a human or a non-human subject.

The inflammatory disease or condition are selected from include rheumatoid arthritis (RA), juvenile rheumatoid arthritis (JRA), osteoarthritis, psoriasis, psoriatic arthritis, ankylosing spondylitis (AS), lupus erythamatosis, multiple sclerosis (MS), asthma, immuno-suppressive therapy (e.g. post transplantation surgery).

The calcitonin used in accordance with this aspect of the invention may be formulated for use by any convenient route. The medicament will usually be supplied as part of a sterile, pharmaceutical composition which will normally include a pharmaceutically acceptable carrier. This pharmaceutical composition may be in any suitable form, (depending upon the desired method of administering it to a patient).

It may be provided in unit dosage form, will generally be provided in a sealed container and may be provided as part of a kit. Such a kit would normally (although not necessarily) include instructions for use. It may include a plurality of said unit dosage forms.

The pharmaceutical composition may be adapted for administration by any appropriate route, for example by the oral (including buccal or sublingual), rectal, nasal, topical (including buccal, sublingual or transdermal), vaginal or parenteral (including subcutaneous, intramuscular, intravenous or intradermal) route. Such compositions may be prepared by any method known in the art of pharmacy, for example by admixing the active ingredient with the carrier(s) or excipient(s) under sterile conditions.

Pharmaceutical compositions adapted for oral administration may be presented as discrete units such as capsules or tablets; as powders or granules; as solutions, syrups or suspensions (in aqueous or non-aqueous liquids; or as edible foams or whips; or as emulsions)

Suitable excipients for tablets or hard gelatine capsules include lactose, maize starch or derivatives thereof, stearic acid or salts thereof.

Suitable excipients for use with soft gelatine capsules include for example vegetable oils, waxes, fats, semi-solid, or liquid polyols etc.

For the preparation of solutions and syrups, excipients which may be used include for example water, polyols and sugars. For the preparation of suspensions oils (e.g. vegetable oils) may be used to provide oil-in-water or water in oil suspensions.

Pharmaceutical compositions adapted for transdermal administration may be presented as discrete patches intended to remain in intimate contact with the epidermis of the recipient for a prolonged period of time. For example, the active ingredient may be delivered from the patch by iontophoresis as generally described in *Pharmaceutical Research,* **3(6)**:318 (1986).

Pharmaceutical compositions adapted for topical administration may be formulated as ointments, creams, suspensions, lotions, powders, solutions, pastes, gels, sprays, aerosols or oils. For infections of the eye or other external tissues, for example mouth and skin, the compositions are preferably applied as a topical ointment or cream. When formulated in an ointment, the active ingredient may be employed with either a paraffinic or a water-miscible ointment base. Alternatively, the active ingredient may be formulated in a cream with an oil-in-water cream base or a water-in-oil base. Pharmaceutical compositions adapted for topical administration to the eye include eye drops wherein the active ingredient is dissolved or suspended in a suitable carrier, especially an aqueous solvent. Pharmaceutical compositions adapted for topical administration in the mouth include lozenges, pastilles and mouth washes. Pharmaceutical compositions adapted for rectal administration may be presented as suppositories or enemas.

Pharmaceutical compositions adapted for nasal administration wherein the carrier is a solid include a coarse powder having a particle size for example in the range 20 to 500 microns which is administered in the manner in which snuff is taken, i.e. by rapid inhalation through the nasal passage from a container of the powder held close up to the nose. Suitable compositions wherein the carrier is a liquid, for administration as a nasal spray or as nasal drops, include aqueous or oil solutions of the active ingredient.

Pharmaceutical compositions adapted for administration by inhalation include fine particle dusts or mists which may be generated by means of various types of metered dose pressurised aerosols, nebulizers or insufflators.

Pharmaceutical compositions adapted for vaginal administration may be presented as pessaries, tampons, creams, gels, pastes, foams or spray formulations.

Pharmaceutical compositions adapted for parenteral administration include aqueous and non-aqueous sterile injection solution which may contain anti-oxidants, buffers, bacteriostats and solutes which render the formulation substantially isotonic with the blood of the intended recipient; and aqueous and non-aqueous sterile suspensions which may include suspending agents and thickening agents. Excipients which may be used for injectable solutions include water, alcohols, polyols, glycerine and vegetable oils, for example. The compositions may be presented in unit-dose or multi-dose containers, for example sealed ampoules and vials, and may be stored in a freeze-dried (lyophilized) condition requiring only the addition of the sterile liquid carried, for example water for injections, immediately prior to use. Extemporaneous injection solutions and suspensions may be prepared from sterile powders, granules and tablets.

The pharmaceutical compositions may contain preserving agents, solubilising agents, stabilising agents, wetting agents, emulsifiers, sweeteners, colourants, odourants, salts (substances of the present invention may themselves be provided in the form of a pharmaceutically acceptable salt), buffers, coating agents or antioxidants. They may also contain therapeutically active agents in addition to the substance of the present invention.

Dosages of the substance of the present invention can vary between wide limits, depending upon the disease or disorder to be treated, the age and condition of the individual to be treated, etc. and a physician will ultimately determine appropriate dosages to be used.

This dosage may be repeated as often as appropriate. If side effects develop the amount and/or frequency of the dosage can be reduced, in accordance with normal clinical practice.

Embodiments in accordance with this aspect of the invention therefore extend to a method of treatment for an inflammatory disease or condition, comprising administration to a subject in need thereof a therapeutically effective amount of a calcitonin in combination with a glucocorticoid to said subject.

The glucocorticoid may be prednisone, prednisolone, dexamethasone, methylprednisolone, budesonide, hydrocortisone, betamethasone, triamcinolone or fludrocoritisone. A preferred glucocorticoid may be prednisolone

An advantage of this aspect of the invention is that it permits a reduction of glucocorticoid dose (thus reducing the effect of secondary osteoporosis). The calcitonin may effectively act as a functional antagonist of glucocorticoid-induced bone loss. The combination of a calcitonin and a glucocorticoid in the treatment of an inflammatory disease or condition provides a synergistic effect over the conventional treatment using a glucocorticoid alone.

The present disclosure also provides a method of treatment for an inflammatory disease or condition, comprising administration to a subject in need thereof a therapeutically effective amount of a calcitonin and of a glucocorticoid to said subject.

This aspect of the invention, therefore also extends to a kit of parts, optionally containing instructions for use, comprising a calcitonin and a glucocorticoid for separate, simultaneous or sequential administration to a subject in need thereof.

According to a second aspect of the invention, there is provided a pharmaceutical composition and a use according to the first aspect of the invention, in which the medicament further comprises an anti-rheumatic drug.

The anti-rheumatic drug may be methotrexate, or sulfasalazine.

The present disclosure also therefore provides a method of treatment for an inflammatory disease or condition, comprising administration to a subject in need thereof a therapeutically effective amount of a calcitonin, a glucocorticoid and an anti-rheumatic drug to said subject.

This aspect of the invention, therefore also extends to a kit of parts, optionally containing instructions for use, comprising a calcitonin, a glucocorticoid and an anti-rheumatic drug for separate, simultaneous or sequential administration to a subject in need thereof.

According to a third aspect of the invention, there is provided a pharmaceutical composition and a use according to the first or to the second aspect of the invention, in which the medicament further comprises an antibody.

The antibody may be monoclonal or polyclonal. The antibodies may be human or from a non-human source. Humanised or fully human antibodies may be preferable according to embodiments of the invention, where the subject to be treated is human.

Polyclonal antibodies can be raised by stimulating their production in a suitable animal host (e.g. a mouse, rat, guinea pig, rabbit, sheep, goat or monkey) when a suitable hapten is injected into the animal. If necessary an adjuvant may be administered together with the substance of the present invention. The antibodies can then be purified by virtue of their binding to a substance of the present invention.

Suitably, the hapten is a substance associated with the onset or progression of an inflammatory disease or condition, such as for example the TNF Receptor (TNF-R).

Monoclonal antibodies can be produced from hybridomas. These can be formed by fusing myeloma cells and spleen cells which produce the desired antibody in order to form an immortal cell line. This is the well known Kohler & Milstein technique (Nature 256 52-55 (1975)). Techniques for producing monoclonal and polyclonal antibodies which bind to a particular protein are now well developed in the art. They are discussed in standard immunology textbooks, for example in Roitt et al, Immunology second edition (1989), Churchill Livingstone, London. Alternatively, phage display technology may be used (McCafferty et al., Nature 348: 552-554 (1990); and as described in WO 92/01047).

In addition to whole antibodies, the term antibody extends to parts thereof which are capable of binding to haptens. Thus the present invention includes antibody fragments and synthetic constructs. Examples of antibody fragments and synthetic constructs are given by Dougall et al in Tibtech 12 372-379 (1994).

Antibody fragments include, for example, Fab, F(ab')₂ and Fv fragments (see Roitt *et al* [*supra*]). Fv fragments can be modified to produce a synthetic construct known as a single chain Fv (scFv) molecule, a single chain antibody fragment (scAb) or a diabody comprising a heavy (V_{H}) chain variable domain connected to a light (V_{L}) chain variable domain which may be bivalent or bispecific. Such fragments may therefore include a peptide linker covalently joining V_{H} and V_{L} regions which contribute to the stability of the molecule. Smaller peptide fragments of the V_{H} domain and V_{L} domain may also be used.

Other synthetic constructs include CDR peptides. These are synthetic peptides comprising antigen binding determinants. Peptide mimetics may also be used. These molecules are usually conformationally restricted organic rings which mimic the structure of a CDR loop and which include antigen-interactive side chains.

Synthetic constructs include chimaeric molecules. Thus, for example, humanised (or primatised) antibodies or derivatives thereof are within the scope of the present invention. An example of a humanised antibody is an antibody having human framework regions, but rodent hypervariable regions. Synthetic constructs also include molecules comprising a covalently linked moiety which provides the molecule with some desirable property in addition to antigen binding. For example the moiety may be a label (e.g. a fluorescent or radioactive label) or a pharmaceutically active agent.

In a preferred embodiment, the antibody is etanercept. Etanercept (ENBREL®) is a dimeric fusion protein consisting of the extracellular ligand-binding portion of the human 75 kilodalton (p75) tumor necrosis factor receptor (TNFR) linked to the Fc portion of human IgG1. The Fc component of etanercept contains the CH2 domain, the CH3 domain and hinge region, but not the CH1 domain of IgG1. Etanercept is produced by recombinant DNA technology in a Chinese hamster ovary (CHO) mammalian cell expression system. It consists of 934 amino acids and has an apparent molecular weight of approximately 150 kilodaltons.

Etanercept has been described in EP 939121, EP 418014, EP 471701, EP 464533, EP 1132471, EP 417563, EP 620739, EP 670730, EP 1239043, WO 91/03553, WO 94/06476, WO 93/19777 and US 5605690.

The present disclosure also therefore provides a method of treatment for an inflammatory disease or condition, comprising administration to a subject in need thereof a therapeutically effective amount of a calcitonin, a glucocorticoid and an antibody to said subject.

This aspect of the invention, therefore also extends to a kit of parts, optionally containing instructions for use, comprising a calcitonin, a glucocorticoid and an antibody for separate, simultaneous or sequential administration to a subject in need thereof.

According to a preferred embodiment of the invention, there is provided a use as described above, of a calcitonin in the preparation of an agent for the treatment of an inflammatory disease or condition, in which the calcitonin is salmon calcitonin or eel calcitonin and the medicament further comprises a glucocorticoid, suitably prednisolone in the treatment of rheumatoid arthritis.

The present invention therefore provides the administration and exploitation of a hitherto unrecognised anti-inflammatory effect of calcitonin. Calcitonin is anti-inflammatory as a single agent but is even more dramatically effective with other anti-inflammatory agents, such as glucocorticoids. Calcitonin acts synergistically with prednisolone. The latter effect is sufficiently strong to lower the required dose of prednisolone by at least five-fold thereby potentially avoiding the deleterious side effects of high dose steroids (e.g. glucocorticoid (GC)-induced osteoporosis).

Tests were performed as described below with administration of calcitonin in an animal model of rheumatoid arthritis in combination with prednisolone, the standard treatment in this model. The focus of calcitonin was to reduce the increased osteoclastic resorption in periarticular bone that is associated with severe rheumatoid arthritis. Unexpectedly, however, calcitonin was found to have a profound anti-inflammatory effect in this model especially when co-administered with prednisolone, where calcitonin potentiated the anti-inflammatory effect of low dose prednisolone. Preferred features for the second and subsequent aspects of the invention are as for the first aspect *mutatis mutandis*.

### EXAMPLES

The present invention will now be further described by way of illustration only with reference to the following Examples, which are not to be construed as being limiting on the invention. In the Examples, reference is also made to a number of drawings in which:
FIGURE 1 shows relationship between CIA and paw swelling (physical symptom associated with joint disruption).
FIGURE 2 shows effect of body weight on treatment
FIGURE 3 shows clinical score
FIGURE 4 shows the degree of disease incidence in the different groups.
FIGURE 5 AND 6 report the same data of Figure 1 arid 3, showing only the CT and Pred+CT groups.
FIGURE 7 shows anti-inflammatory activity of calcitonin alone.

### Experiment 1: Study of effect of treatment protocol on experimentally-induced arthritis

*Collagen II*-*induced arthritis (CIA).* Female Lewis rats (150 ± 20 g body weight; Harlan UK Ltd Bicester, Oxfordshire, England) were fed on a standard chow pellet diet and had free access to water and maintained on a 12 h light/dark cycles. Animal work was carried out under licence from the Home Office in accordance with the Animals (Scientific Procedures) Act, 1986. Bovine nasal collagen II (4 mg/ml; Sigma-Aldrich Ltd, Poole, UK) was dissolved in acetic acid (0.01M) then emulsified with the same volume of ice-cold Freund's incomplete adjuvant (Sigma-Aldrich). On day 0, rats were anaesthetised with halothane, the base of the tail shaved and injected intradermally with collagen II/adjuvant suspension (400 µg collagen II per rat). The first signs of arthritis were evident between days 11 and 13, with maximal inflammation observed at day 18-21.

CIA-induced inflammation was confined to ankle joints and footpads of the hind legs (with digit involvement in severe cases). Hind ankles were scored clinically on an arbitrary scale, ranging from 0 (no inflammation) to three (severe inflammation, involving ankles, footpads and digits). In addition, between day 0 and 18 hind paw volumes were measured using a plethysmometer (Ugo Basile, Milan, Italy) and values averaged to give a measurement of inflammation for each animal.

Salmon CT was dissolved in PBS with 0.1% BSA and given daily at the dose of 2µg/kg per rat. Prednisolone (prednisolone 21-hemisuccinate, sodium salt) was dissolved PBS with 0.1% BSA and given i.p. at doses of 0.6 and 3 mg/kg (corresponding to 1.2 and 6.2 µmol/kg), alone or with CT. Control treatments consisted in PBS with 0.1% BSA.

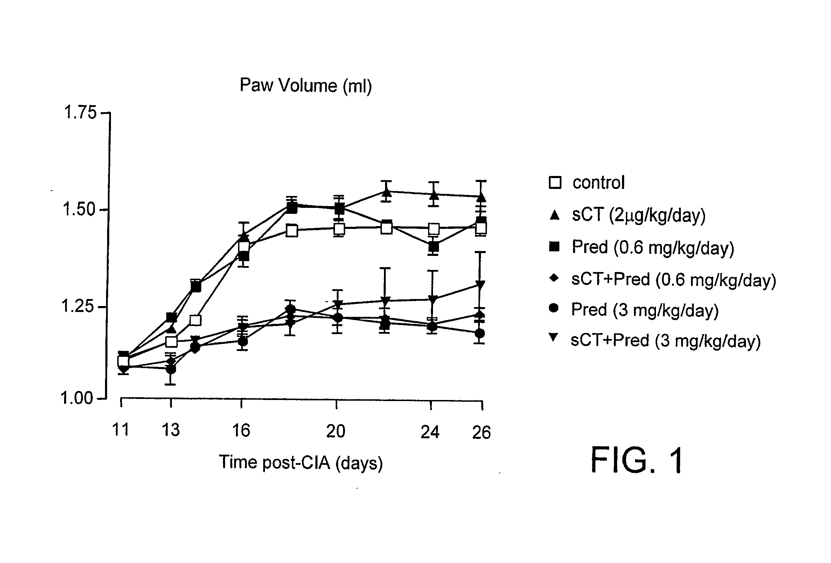

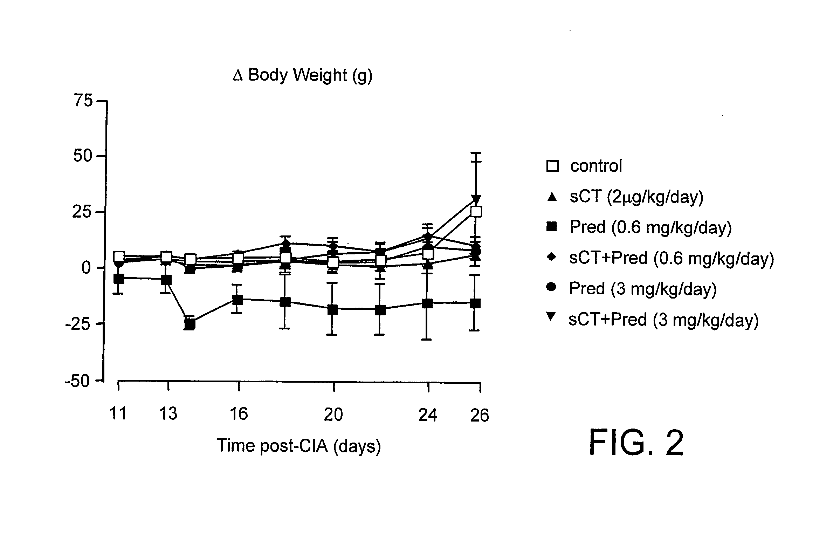

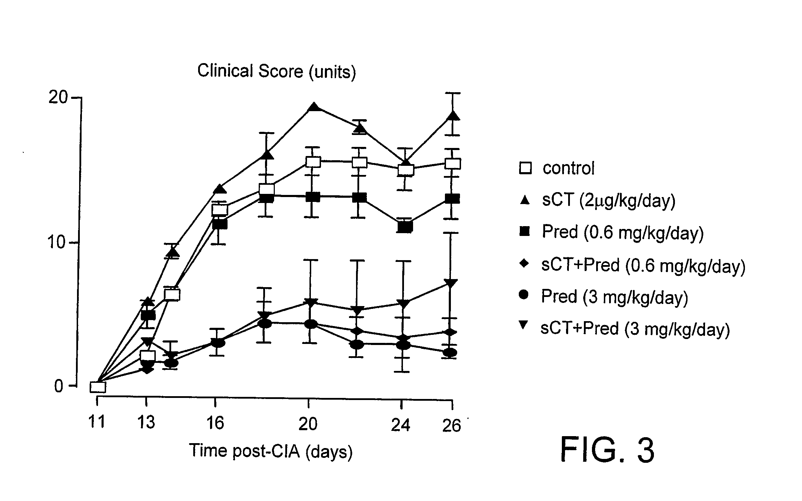

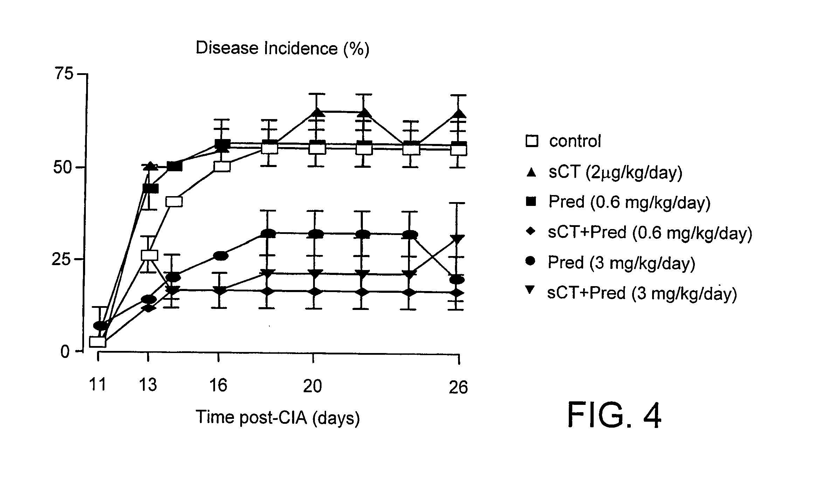

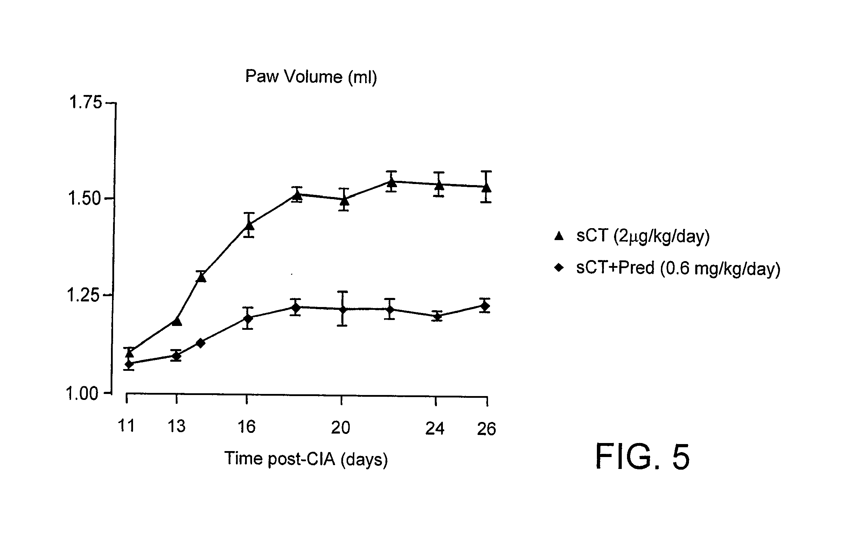

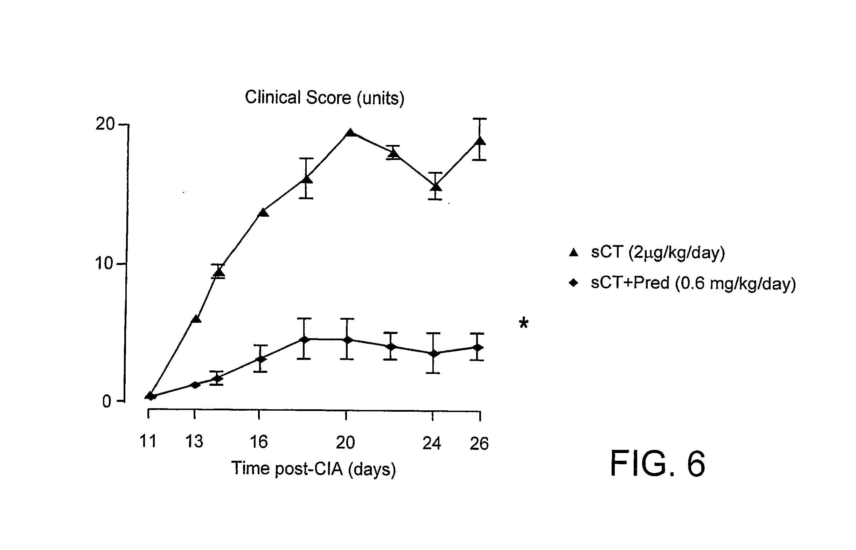

Figure 1 shows that CIA was characterised by marked increase in paw swelling (physical symptom associated with joint disruption). Treatment with CT (or with the low dose of prednisolone (Pred), did not modify paw swelling. However, the combination CT + low dose prednisolone resulted in marked attenuation of paw swelling (blue diamonds). This effect was no longer evident when a full anti-inflammatory dose of the GC was applied. The effects described in Figure 1 were not secondary to alteration in the health status of the animals, as body weight was not different across the different treatments (Figure 2). Figure 3 shows that the changes in paw swelling were also reflected in alterations in the clinical score. Finally, Figure 4 illustrates the degree of disease incidence in the different groups. Again, the protective effect of the CT + low dose prednisolone is remarkable. In all these figures, values are mean ± s.e.mean of n=10 rats per group. Statistical difference to a P value less than 0.05 was attained by the two groups with the high dose prednisolone (with or without CT) as well as by the CT + low dose prednisolone group (blue diamonds). Figure 5 and 6 report the same data of Figure 1 and 3, showing only the CT and Pred+CT groups.

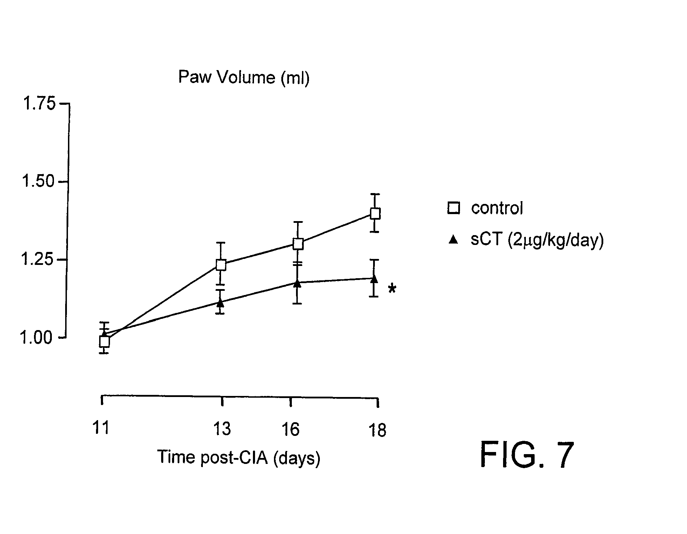

In previous older experiments, treatment with calcitonin (0.5 µg/rat) produced a mild anti-inflammatory action on its own (see Figure 7).

### Conclusion

These results show that the combined treatment of calcitonin with a GC has anti-inflammatory properties in the collagen-induced arthritis model and would allow reduction of the dose of the GC to be effective. It is also worth noting the anti-inflammatory effects produced by this dose of CT which inhibited between 5 and 20% the arthritic response.

## Claims

1. Calcitonin for use in the treatment of an inflammatory disease or condition in combination with a glucocorticoid, wherein the inflammatory disease or condition is selected from the group consisting of arthritis, rheumatoid arthritis (RA), juvenile rheumatoid arthritis (JRA), osteoarthritis, psoriasis, psoriatic arthritis, ankylosing spondylitis (AS), lupus erythamatosis, multiple sclerosis (MS), asthma, and immune-suppressive therapy.

2. Calcitonin for use according to claim 1, in which the glucocorticoid is selected from the group consisting of prednisolone, dexamethasone, methylprednisolone, budesonide, hydrocortisone, betamethasone, triamcinolone, fludrocortisone and prednisone.

3. Calcitonin for use according to claim 1 or claim 2, in which the calcitonin is for use in further combination with an anti-rheumatic drug.

4. Calcitonin for use according to claim 3, in which the anti-rheumatic drug is methotrexate.

5. Calcitonin for use according to any one of claims 1 to 4, in which the calcitonin is for use in further combination with an antibody raised against the TNF-Receptor (TNF-R).

6. Calcitonin for use according to claim 5, in which the antibody is a monoclonal antibody.

7. Calcitonin for use according to claim 6, in which the monoclonal antibody is a monoclonal antibody fusion protein.

8. Calcitonin for use as claimed in claim 7, in which the monoclonal antibody fusion protein is etanercept, wherein etanercept is a dimeric fusion protein consisting of the extracellular ligand-binding portion of the 75 kilodalton (p75) tumour necrosis factor receptor (TNFR) linked to the Fc portion of the human IgG1.

9. Calcitonin for use according to any one of claims 1 to 8 in which the calcitonin is selected from the group consisting of human, salmon, porcine, bovine, eel or chicken calcitonin.

## Patentansprüche

1. Calcitonin zur Verwendung in der Behandlung einer Entzündungserkrankung oder eines Entzündungszustandes in Kombination mit einem Glucocorticoid, wobei die Entzündungserkrankung oder der Entzündungszustand ausgewählt ist aus der Gruppe bestehend aus Arthritis, rheumatoider Arthritis (RA), juveniler rheumatoider Arthritis (JRA), Osteoarthritis, Psoriasis, psoriatischer Arthritis, ankylosierender Spondylitis (AS), Lupus erythematodes, Multipler Sklerose (MS), Asthma und immunsuppressiver Therapie.

2. Calcitonin zur Verwendung nach Anspruch 1, wobei das Glucocorticoid ausgewählt ist aus der Gruppe bestehend aus Prednisolon, Dexamethason, Methylprednisolon, Budesonid, Hydrocortison, Betamethason, Triamcinolon, Fludrocortison und Prednison.

3. Calcitonin zur Verwendung nach Anspruch 1 oder Anspruch 2, wobei Calcitonin zur Verwendung in weiterer Kombination mit einem antirheumatischen Arzneimittel dient.

4. Calcitonin zur Verwendung nach Anspruch 3, wobei das antirheumatische Arzneimittel Methotrexat ist.

5. Calcitonin zur Verwendung nach einem der Ansprüche 1 bis 4, wobei das Calcitonin zur Verwendung in weiterer Kombination mit einem Antikörper dient, der gegen den TNF-Rezeptor (TNF-R) gebildet wird.

6. Calcitonin zur Verwendung nach Anspruch 5, wobei der Antikörper ein monoklonaler Antikörper ist.

7. Calcitonin zur Verwendung nach Anspruch 6, wobei der monoklonale Antikörper ein monoklonaler Antikörper-Fusionsprotein ist.

8. Calcitonin zur Verwendung nach Anspruch 7, wobei das monoklonaler Antikörper-Fusionsprotein Etanercept ist, wobei Etanercept ein dimeres Fusionsprotein ist, das aus dem extrazellulären Ligandenbindungsteil des 75 Kilodalton (p75) Tumornekrosefaktorrezeptors (TNFR), gebunden an den Fc-Teil des humanen IgG1, besteht.

9. Calcitonin zur Verwendung nach einem der Ansprüche 1 bis 8, wobei das Calcitonin ausgewählt ist aus der Gruppe bestehend aus Human-, Lachs-, Schweine-, Rinder-, Aal- oder Hühner-Calcitonin.

## Revendications

1. Calcitonine utilisable pour le traitement d'une maladie ou affection inflammatoire en combinaison avec un glucocorticoïde, dans laquelle la maladie ou affection inflammatoire est choisie dans le groupe constitué de l'arthrite, la polyarthrite rhumatoïde (RA), la polyarthrite rhumatoïde juvénile (JRA), l'ostéoarthrite, le psoriasis, l'arthrite psoriasique, la spondylarthrite ankylosante (AS), le lupus érythémateux, la sclérose en plaques (MS), l'asthme, et une thérapie immunosuppressive.

2. Calcitonine utilisable selon la revendication 1, dans laquelle le glucocorticoïde est choisi dans le groupe constitué de prednisolone, dexaméthasone, méthylprednisolone, budésonide, hydrocortisone, bétaméthasone, triamcinolone, fludrocortisone et prednisone.

3. Calcitonine utilisable selon la revendication 1 ou la revendication 2, où la calcitonine est destinée à être utilisée en combinaison supplémentaire avec un médicament antirhumatismal.

4. Calcitonine utilisable selon la revendication 3, dans laquelle le médicament anti-rhumatismal est le méthotrexate.

5. Calcitonine utilisable selon l'une quelconque des revendications 1 à 4, où la calcitonine est destinée à être utilisée en combinaison supplémentaire avec un anticorps dressé contre le récepteur de TNF (TNF-R).

6. Calcitonine utilisable selon la revendication 5, dans laquelle l'anticorps est un anticorps monoclonal.

7. Calcitonine utilisable selon la revendication 6, dans laquelle l'anticorps monoclonal est une protéine de fusion d'anticorps monoclonal.

8. Calcitonine utilisable selon la revendication 7, dans laquelle la protéine de fusion d'anticorps monoclonal est l'étanercept, dans laquelle l'étanercept est une protéine de fusion dimère constituée de la partie de liaison de ligand extracellulaire du récepteur de facteur de nécrose tumorale (TNFR) de 75 kilodaltons (p75) liée à la partie Fc de l'IgG1 humain.

9. Calcitonine utilisable selon l'une quelconque des revendications 1 à 8, où la calcitonine est choisie dans le groupe constitué de calcitonine d'humain, de saumon, de porcin, de bovin, d'anguille ou de poulet.
